# EUROPÄISCHE PATENTSCHRIFT

(11) **EP 1 745 063 B1**
(45) Veröffentlichungstag und Bekanntmachung des Hinweises auf die Patenterteilung: **12.11.2008**
(21) Anmeldenummer: 05739879.4
(22) Anmeldetag: 12.05.2005
(51) Int. Cl.: C07K 1/04, C07H 21/00, G01N 33/68

(54) **VERFAHREN ZUR HERSTELLUNG VON CHEMISCHEN MIKROARRAYS**
METHOD FOR PRODUCING CHEMICAL MICROARRAYS
PROCEDE DE FABRICATION DE MICRORESEAUX CHIMIQUES

(30) Priorität: 13.05.2004 DE 102004023906
(43) Veröffentlichungstag der Anmeldung: 24.01.2007
(73) Patentinhaber: Helmholtz-Zentrum für Infektionsforschung GmbH, 38124 Braunschweig (DE)
(72) Erfinder: RONALD, Frank, 38527 Meine (DE); BEUTLING, Ulrike, 38124 Braunschweig (DE); SWISTOWSKI, Andrzej, 38126 Braunschweig (DE); DIKMANS, Antonius, 38106 Braunschweig (DE); GUPTE, Varsha, 38118 Braunschweig (DE); THIELE, Sabine, 38106 Braunschweig (DE)
(74) Vertreter: Forstmeyer, Dietmar
(86) Internationale Anmeldenummer: PCT/EP2005/005189
(87) Internationale Veröffentlichungsnummer: WO 2005/111061

(56) Entgegenhaltungen:
- WO-A-03/074722
- BIOCONJUGATE CHEMISTRY., Bd. 12, 2001, Seiten 346-353, XP002339773 US AMERICAN CHEMICAL SOCIETY, WASHINGTON.
- MOLECULAR DIVERSITY., Bd. 8, Nr. 2, September 2004 (2004-09), Seiten 301-310, XP002339774 NL ESCOM SCIENCE PUBLISHERS, LEIDEN. in der Anmeldung erwähnt
- SCHENA M.: 'Protein microarrays', 2005, JONES & BARTLETT, BOSTON * Seite 268 - Seite 269 *

### SEQUENCE LISTING

<110> Gesellschaft für Biotechnologische Forschung mbH (GBF)
<120> Verfahren zur Herstellung von chemischen Mikroarrays
<130> 14978-GBF
<160> 125
<170> PatentIn version 3.1
<210> 1
   <211> 6
   <212> PRT
   <213> Artificial Sequence
<220>
   <223> Synthetische Peptidsequence
<400> 1
   Ala Tyr Gly Lys Tyr Glu
   1               5
<210> 2
   <211> 6
   <212> PRT
   <213> Artificial Sequence
<220>
   <223> Synthetische Peptidsequenz
<400> 2
   Cys Tyr Gly Lys Tyr Glu
   1               5
<210> 3
   <211> 6
   <212> PRT
   <213> Artificial Sequence
<220>
   <223> Synthetische Peptidsequenz
<400> 3
   Asp Tyr Gly Lys Tyr Glu
   1               5
<210> 4
   <211> 6
   <212> PRT
   <213> Artificial Sequence
<220>
   <223> Synthetische Peptidsequenz
<400> 4
   Glu Tyr Gly Lys Tyr Glu
   1               5
<210> 5
   <211> 6
   <212> PRT
   <213> Artificial Sequence
<220>
   <223> Synthetische Peptidsequenz
<400> 5
   Phe Tyr Gly Lys Tyr Glu
   1               5
<210> 6
   <211> 6
   <212> PRT
   <213> Artificial Sequence
<220>
   <223> Synthetische Peptidsequenz
<400> 6
   Gly Tyr Gly Lys Tyr Glu
   1 5
<210> 7
   <211> 6
   <212> PRT
   <213> Artificial Sequence
<220>
   <223> Synthetische Peptidsequenz
<400> 7
   His Tyr Gly Lys Tyr Glu
   1 5
<210> 8
   <211> 6
   <212> PRT
   <213> Artificial Sequence
<220>
   <223> Synthetische Peptidsequenz
<400> 8
   Ile Tyr Gly Lys Tyr Glu
   1 5
<210> 9
   <211> 6
   <212> PRT
   <213> Artificial Sequence
<220>
   <223> Synthetische Peptidsequenz
<400> 9
   Lys Tyr Gly Lys Tyr Glu
   1 5
<210> 10
   <211> 6
   <212> PRT
   <213> Artificial Sequence
<220>
   <223> Synthetische Peptidsequenz
<400> 10
   Leu Tyr Gly Lys Tyr Glu
   1 5
<210> 11
   <211> 6
   <212> PRT
   <213> Artificial Sequence
<220>
   <223> Synthetische Peptidsequenz
<400> 11
   Met Tyr Gly Lys Tyr Glu
   1 5
<210> 12
   <211> 6
   <212> PRT
   <213> Artificial Sequence
<220>
   <223> Synthetische Peptidsequenz
<400> 12
   Asn Tyr Gly Lys Tyr Glu
   1 5
<210> 13
   <211> 6
   <212> PRT
   <213> Artificial Sequence
<220>
   <223> Synthetische Peptidsequenz
<400> 13
   Pro Tyr Gly Lys Tyr Glu
   1 5
<210> 14
   <211> 6
   <212> PRT
   <213> Artificial Sequence
<220>
   <223> Synthetische Peptidsequenz
<400> 14
   Gln Tyr Gly Lys Tyr Glu
   1 5
<210> 15
   <211> 6
   <212> PRT
   <213> Artificial Sequence
<220>
   <223> Synthetische Peptidsequenz
<400> 15
   Arg Tyr Gly Lys Tyr Glu
   1 5
<210> 16
   <211> 6
   <212> PRT
   <213> Artificial Sequence
<220>
   <223> Synthetische Peptidsequenz
<400> 16
   Ser Tyr Gly Lys Tyr Glu
   1 5
<210> 17
   <211> 6
   <212> PRT
   <213> Artificial Sequence
<220>
   <223> Synthetische Peptidsequenz
<400> 17
   Thr Tyr Gly Lys Tyr Glu
   1 5
<210> 18
   <211> 6
   <212> PRT
   <213> Artificial Sequence
<220>
   <223> Synthetische Peptidsequenz
<400> 18
   Val Tyr Gly Lys Tyr Glu
   1 5
<210> 19
   <211> 6
   <212> PRT
   <213> Artificial Sequence
<220>
   <223> Synthetische Peptidsequenz
<400> 19
   Trp Tyr Gly Lys Tyr Glu
   1 5
<210> 20
   <211> 6
   <212> PRT
   <213> Artificial Sequence
<220>
   <223> Synthetische Peptidsequenz
<400> 20
   Tyr Tyr Gly Lys Tyr Glu
   1 5
<210> 21
   <211> 6
   <212> PRT
   <213> Artificial Sequence
<220>
   <223> Synthetische Peptidsequenz
<400> 21
   Asn Ala Gly Lys Tyr Glu
   1 5
<210> 22
   <211> 6
   <212> PRT
   <213> Artificial Sequence
<220>
   <223> Synthetische Peptidsequenz
<400> 22
   Asn Cys Gly Lys Tyr Glu
   1               5
<210> 23
   <211> 6
   <212> PRT
   <213> Artificial Sequence
<220>
   <223> Synthetische Peptidsequenz
<400> 23
   Asn Asp Gly Lys Tyr Glu
   1               5
<210> 24
   <211> 6
   <212> PRT
   <213> Artificial Sequence
<220>
   <223> Synthetische Peptidsequenz
<400> 24
   Asn Glu Gly Lys Tyr Glu
   1               5
<210> 25
   <211> 6
   <212> PRT
   <213> Artificial Sequence
<220>
   <223> Synthetische Peptidsequenz
<400> 25
   Asn Phe Gly Lys Tyr Glu
   1               5
<210> 26
   <211> 6
   <212> PRT
   <213> Artificial Sequence
<220>
   <223> Synthetische Peptidsequenz
<400> 26
   Asn Gly Gly Lys Tyr Glu
   1               5
<210> 27
   <211> 6
   <212> PRT
   <213> Artificial Sequence
<220>
   <223> Synthetische Peptidsequenz
<400> 27
   Asn His Gly Lys Tyr Glu
   1               5
<210> 28
   <211> 6
   <212> PRT
   <213> Artificial Sequence
<220>
   <223> Synthetische Peptidsequenz
<400> 28
   Asn Ile Gly Lys Tyr Glu
   1               5
<210> 29
   <211> 6
   <212> PRT
   <213> Artificial Sequence
<220>
   <223> Synthetische Peptidsequenz
<400> 29
   Asn Lys Gly Lys Tyr Glu
   1 5
<210> 30
   <211> 6
   <212> PRT
   <213> Artificial Sequence
<220>
   <223> Synthetische Peptidsequenz
<400> 30
   Asn Leu Gly Lys Tyr Glu
   1 5
<210> 31
   <211> 6
   <212> PRT
   <213> Artificial Sequence
<220>
   <223> Synthetische Peptidsequenz
<400> 31
   Asn Met Gly Lys Tyr Glu
   1 5
<210> 32
   <211> 6
   <212> PRT
   <213> Artificial Sequence
<220>
   <223> Synthetische Peptidsequenz
<400> 32
   Asn Asn Gly Lys Tyr Glu
   1 5
<210> 33
   <211> 6
   <212> PRT
   <213> Artificial Sequence
<220>
   <223> Synthetische Peptidsequenz
<400> 33
   Asn Pro Gly Lys Tyr Glu
   1 5
<210> 34
   <211> 6
   <212> PRT
   <213> Artificial Sequence
<220>
   <223> Synthetische Peptidsequenz
<400> 34
   Asn Gln Gly Lys Tyr Glu
   1 5
<210> 35
   <211> 6
   <212> PRT
   <213> Artificial Sequence
<220>
   <223> Synthetische Peptidsequenz
<400> 35
   Asn Arg Gly Lys Tyr Glu
   1 5
<210> 36
   <211> 6
   <212> PRT
   <213> Artificial Sequence
<220>
   <223> Synthetische Peptidsequenz
<400> 36
   Asn Ser Gly Lys Tyr Glu
   1 5
<210> 37
   <211> 6
   <212> PRT
   <213> Artificial Sequence
<220>
   <223> Synthetische Peptidsequenz
<400> 37
   Asn Thr Gly Lys Tyr Glu
   1 5
<210> 38
   <211> 6
   <212> PRT
   <213> Artificial Sequence
<220>
   <223> Synthetische Peptidsequenz
<400> 38
   Asn Val Gly Lys Tyr Glu
   1 5
<210> 39
   <211> 6
   <212> PRT
   <213> Artificial Sequence
<220>
   <223> Synthetische Peptidsequenz
<400> 39
   Asn Trp Gly Lys Tyr Glu
   1 5
<210> 40
   <211> 6
   <212> PRT
   <213> Artificial Sequence
<220>
   <223> Synthetische Peptidsequenz
<400> 40
   Asn Tyr Gly Lys Tyr Glu
   1 5
<210> 41
   <211> 6
   <212> PRT
   <213> Artificial Sequence
<220>
   <223> Synthetische Peptidsequenz
<400> 41
   Asn Tyr Ala Lys Tyr Glu
   1 5
<210> 42
   <211> 6
   <212> PRT
   <213> Artificial Sequence
<220>
   <223> Synthetische Peptidsequenz
<400> 42
   Asn Tyr Cys Lys Tyr Glu
   1 5
<210> 43
   <211> 6
   <212> PRT
   <213> Artificial Sequence
<220>
   <223> Synthetische Peptidsequenz
<400> 43
   Asn Tyr Asp Lys Tyr Glu
   1 5
<210> 44
   <211> 6
   <212> PRT
   <213> Artificial Sequence
<220>
   <223> Synthetische Peptidsequenz
<400> 44
   Asn Tyr Glu Lys Tyr Glu
   1 5
<210> 45
   <211> 6
   <212> PRT
   <213> Artificial Sequence
<220>
   <223> Synthetische Peptidsequenz
<400> 45
   Asn Tyr Phe Lys Tyr Glu
   1 5
<210> 46
   <211> 6
   <212> PRT
   <213> Artificial Sequence
<220>
   <223> Synthetische Peptidsequenz
<400> 46
   Asn Tyr Gly Lys Tyr Glu
   1 5
<210> 47
   <211> 6
   <212> PRT
   <213> Artificial Sequence
<220>
   <223> Synthetische Peptidsequenz
<400> 47
   Asn Tyr His Lys Tyr Glu
   1 5
<210> 48
   <211> 6
   <212> PRT
   <213> Artificial Sequence
<220>
   <223> Synthetische Peptidsequenz
<400> 48
   Asn Tyr Ile Lys Tyr Glu
   1 5
<210> 49
   <211> 6
   <212> PRT
   <213> Artificial Sequence
<220>
   <223> Synthetische Peptidsequenz
<400> 49
   Asn Tyr Lys Lys Tyr Glu
   1 5
<210> 50
   <211> 6
   <212> PRT
   <213> Artificial Sequence
<220>
   <223> Synthetische Peptidsequenz
<400> 50
   Asn Tyr Leu Lys Tyr Glu
   1 5
<210> 51
   <211> 6
   <212> PRT
   <213> Artificial Sequence
<220>
   <223> Synthetische Peptidsequenz
<400> 51
   Asn Tyr Met Lys Tyr Glu
   1 5
<210> 52
   <211> 6
   <212> PRT
   <213> Artificial Sequence
<220>
   <223> Synthetische Peptidsequenz
<400> 52
   Asn Tyr Asn Lys Tyr Glu
   1 5
<210> 53
   <211> 6
   <212> PRT
   <213> Artificial Sequence
<220>
   <223> Synthetische Peptidsequenz
<400> 53
   Asn Tyr Pro Lys Tyr Glu
   1 5
<210> 54
   <211> 6
   <212> PRT
   <213> Artificial Sequence
<220>
   <223> Synthetische Peptidsequenz
<400> 54
   Asn Tyr Gln Lys Tyr Glu
   1 5
<210> 55
   <211> 6
   <212> PRT
   <213> Artificial Sequence
<220>
   <223> Synthetische Peptidsequenz
<400> 55
   Asn Tyr Arg Lys Tyr Glu
   1 5
<210> 56
   <211> 6
   <212> PRT
   <213> Artificial Sequence
<220>
   <223> Synthetische Peptidsequenz
<400> 56
   Asn Tyr Ser Lys Tyr Glu
   1 5
<210> 57
   <211> 6
   <212> PRT
   <213> Artificial Sequence
<220>
   <223> Synthetische Peptidsequenz
<400> 57
   Asn Tyr Thr Lys Tyr Glu
   1 5
<210> 58
   <211> 6
   <212> PRT
   <213> Artificial Sequence
<220>
   <223> Synthetische Peptidsequenz
<400> 58
   Asn Tyr Val Lys Tyr Glu
   1 5
<210> 59
   <211> 6
   <212> PRT
   <213> Artificial Sequence
<220>
   <223> Synthetische Peptidsequenz
<400> 59
   Asn Tyr Trp Lys Tyr Glu
   1 5
<210> 60
   <211> 6
   <212> PRT
   <213> Artificial Sequence
<220>
   <223> Synthetische Peptidsequenz
<400> 60
   Asn Tyr Tyr Lys Tyr Glu
   1 5
<210> 61
   <211> 6
   <212> PRT
   <213> Artificial Sequence
<220>
   <223> Synthetische Peptidsequenz
<400> 61
   Asn Tyr Gly Ala Tyr Glu
   1               5
<210> 62
   <211> 6
   <212> PRT
   <213> Artificial Sequence
<220>
   <223> Synthetische Peptidsequenz
<400> 62
   Asn Tyr Gly Cys Tyr Glu
   1               5
<210> 63
   <211> 6
   <212> PRT
   <213> Artificial Sequence
<220>
   <223> Synthetische Peptidsequenz
<400> 63
   Asn Tyr Gly Asp Tyr Glu
   1               5
<210> 64
   <211> 6
   <212> PRT
   <213> Artificial Sequence
<220>
   <223> Synthetische Peptidsequenz
<400> 64
   Asn Tyr Gly Glu Tyr Glu
   1               5
<210> 65
   <211> 6
   <212> PRT
   <213> Artificial Sequence
<220>
   <223> Synthetische Peptidsequenz
<400> 65
   Asn Tyr Gly Phe Tyr Glu
   1               5
<210> 66
   <211> 6
   <212> PRT
   <213> Artificial Sequence
<220>
   <223> Synthetische Peptidsequenz
<400> 66
   Asn Tyr Gly Gly Tyr Glu
   1               5
<210> 67
   <211> 6
   <212> PRT
   <213> Artificial Sequence
<220>
   <223> Synthetische Peptidsequenz
<400> 67
   Asn Tyr Gly His Tyr Glu
   1               5
<210> 68
   <211> 6
   <212> PRT
   <213> Artificial Sequence
<220>
   <223> Synthetische Peptidsequenz
<400> 68
   Asn Tyr Gly Ile Tyr Glu
   1               5
<210> 69
   <211> 6
   <212> PRT
   <213> Artificial Sequence
<220>
   <223> Synthetische Peptidsequenz
<400> 69
   Asn Tyr Gly Lys Tyr Glu
   1 5
<210> 70
   <211> 6
   <212> PRT
   <213> Artificial Sequence
<220>
   <223> Synthetische Peptidsequenz
<400> 70
   Asn Tyr Gly Leu Tyr Glu
   1 5
<210> 71
   <211> 6
   <212> PRT
   <213> Artificial Sequence
<220>
   <223> Synthetische Peptidsequenz
<400> 71
   Asn Tyr Gly Met Tyr Glu
   1 5
<210> 72
   <211> 6
   <212> PRT
   <213> Artificial Sequence
<220>
   <223> Synthetische Peptidsequenz
<400> 72
   Asn Tyr Gly Asn Tyr Glu
   1 5
<210> 73
   <211> 6
   <212> PRT
   <213> Artificial Sequence
<220>
   <223> Synthetische Peptidsequenz
<400> 73
   Asn Tyr Gly Pro Tyr Glu
   1 5
<210> 74
   <211> 6
   <212> PRT
   <213> Artificial Sequence
<220>
   <223> Synthetische Peptidsequenz
<400> 74
   Asn Tyr Gly Gln Tyr Glu
   1 5
<210> 75
   <211> 6
   <212> PRT
   <213> Artificial Sequence
<220>
   <223> Synthetische Peptidsequenz
<400> 75
   Asn Tyr Gly Arg Tyr Glu
   1 5
<210> 76
   <211> 6
   <212> PRT
   <213> Artificial Sequence
<220>
   <223> Synthetische Peptidsequenz
<400> 76
   Asn Tyr Gly Ser Tyr Glu
   1 5
<210> 77
   <211> 6
   <212> PRT
   <213> Artificial Sequence
<220>
   <223> Synthetische Peptidsequenz
<400> 77
   Asn Tyr Gly Thr Tyr Glu
   1 5
<210> 78
   <211> 6
   <212> PRT
   <213> Artificial Sequence
<220>
   <223> Synthetische Peptidsequenz
<400> 78
   Asn Tyr Gly Val Tyr Glu
   1 5
<210> 79
   <211> 6
   <212> PRT
   <213> Artificial Sequence
<220>
   <223> Synthetische Peptidsequenz
<400> 79
   Asn Tyr Gly Trp Tyr Glu
   1 5
<210> 80
   <211> 6
   <212> PRT
   <213> Artificial Sequence
<220>
   <223> Synthetische Peptidsequenz
<400> 80
   Asn Tyr Gly Tyr Tyr Glu
   1 5
<210> 81
   <211> 6
   <212> PRT
   <213> Artificial Sequence
<220>
   <223> Synthetische Peptidsequenz
<400> 81
   Asn Tyr Gly Lys Ala Glu
   1 5
<210> 82
   <211> 6
   <212> PRT
   <213> Artificial Sequence
<220>
   <223> Synthetische Peptidsequenz
<400> 82
   Asn Tyr Gly Lys Cys Glu
   1 5
<210> 83
   <211> 6
   <212> PRT
   <213> Artificial Sequence
<220>
   <223> Synthetische Peptidsequenz
<400> 83
   Asn Tyr Gly Lys Asp Glu
   1 5
<210> 84
   <211> 6
   <212> PRT
   <213> Artificial Sequence
<220>
   <223> Synthetische Peptidsequenz
<400> 84
   Asn Tyr Gly Lys Glu Glu
   1 5
<210> 85
   <211> 6
   <212> PRT
   <213> Artificial Sequence
<220>
   <223> Synthetische Peptidsequenz
<400> 85
   Asn Tyr Gly Lys Phe Glu
   1 5
<210> 86
   <211> 6
   <212> PRT
   <213> Artificial Sequence
<220>
   <223> Synthetische Peptidsequenz
<400> 86
   Asn Tyr Gly Lys Gly Glu
   1 5
<210> 87
   <211> 6
   <212> PRT
   <213> Artificial Sequence
<220>
   <223> Synthetische Peptidsequenz
<400> 87
   Asn Tyr Gly Lys His Glu
   1 5
<210> 88
   <211> 6
   <212> PRT
   <213> Artificial Sequence
<220>
   <223> Synthetische Peptidsequenz
<400> 88
   Asn Tyr Gly Lys Ile Glu
   1 5
<210> 89
   <211> 6
   <212> PRT
   <213> Artificial Sequence
<220>
   <223> Synthetische Peptidsequenz
<400> 89
   Asn Tyr Gly Lys Lys Glu
   1 5
<210> 90
   <211> 6
   <212> PRT
   <213> Artificial Sequence
<220>
   <223> Synthetische Peptidsequenz
<400> 90
   Asn Tyr Gly Lys Leu Glu
   1 5
<210> 91
   <211> 6
   <212> PRT
   <213> Artificial Sequence
<220>
   <223> Synthetische Peptidsequenz
<400> 91
   Asn Tyr Gly Lys Met Glu
   1 5
<210> 92
   <211> 6
   <212> PRT
   <213> Artificial
<400> 92
   Asn Tyr Gly Lys Asn Glu
   1 5
<210> 93
   <211> 6
   <212> PRT
   <213> Artificial Sequence
<220>
   <223> Synthetische Peptidsequenz
<400> 93
   Asn Tyr Gly Lys Pro Glu
   1 5
<210> 94
   <211> 6
   <212> PRT
   <213> Artificial Sequence
<220>
   <223> Synthetische Peptidsequenz
<400> 94
   Asn Tyr Gly Lys Gln Glu
   1 5
<210> 95
   <211> 6
   <212> PRT
   <213> Artificial Sequence
<220>
   <223> Synthetische Peptidsequenz
<400> 95
   Asn Tyr Gly Lys Arg Glu
   1 5
<210> 96
   <211> 6
   <212> PRT
   <213> Artificial Sequence
<220>
   <223> Synthetische Peptidsequenz
<400> 96
   Asn Tyr Gly Lys Ser Glu
   1 5
<210> 97
   <211> 6
   <212> PRT
   <213> Artificial Sequence
<220>
   <223> Synthetische Peptidsequenz
<400> 97
   Asn Tyr Gly Lys Thr Glu
   1 5
<210> 98
   <211> 6
   <212> PRT
   <213> Artificial Sequence
<220>
   <223> Synthetische Peptidsequenz
<400> 98
   Asn Tyr Gly Lys Val Glu
   1 5
<210> 99
   <211> 6
   <212> PRT
   <213> Artificial Sequence
<220>
   <223> Synthetische Peptidsequenz
<400> 99
   Asn Tyr Gly Lys Trp Glu
   1 5
<210> 100
   <211> 6
   <212> PRT
   <213> Artificial Sequence
<220>
   <223> Synthetische Peptidsequenz
<400> 100
   Asn Tyr Gly Lys Tyr Glu
   1 5
<210> 101
   <211> 6
   <212> PRT
   <213> Artificial Sequence
<220>
   <223> Synthetische Peptidsequenz
<400> 101
   Asn Tyr Gly Lys Tyr Ala
   1 5
<210> 102
   <211> 6
   <212> PRT
   <213> Artificial Sequence
<220>
   <223> Synthetische Peptidsequenz
<400> 102
   Asn Tyr Gly Lys Tyr Cys
   1 5
<210> 103
   <211> 6
   <212> PRT
   <213> Artificial Sequence
<220>
   <223> Synthetische Peptidsequenz
<400> 103
   Asn Tyr Gly Lys Tyr Asp
   1 5
<210> 104
   <211> 6
   <212> PRT
   <213> Artificial Sequence
<220>
   <223> Synthetische Peptidsequenz
<400> 104
   Asn Tyr Gly Lys Tyr Glu
   1 5
<210> 105
   <211> 6
   <212> PRT
   <213> Artificial Sequence
<220>
   <223> Synthetische Peptidsequenz
<400> 105
   Asn Tyr Gly Lys Tyr Phe
   1 5
<210> 106
   <211> 6
   <212> PRT
   <213> Artificial Sequence
<220>
   <223> Synthetische Peptidsequenz
<400> 106
   Asn Tyr Gly Lys Tyr Gly
   1 5
<210> 107
   <211> 6
   <212> PRT
   <213> Artificial Sequence
<220>
   <223> Synthetische Peptidsequenz
<400> 107
   Asn Tyr Gly Lys Tyr His
   1 5
<210> 108
   <211> 6
   <212> PRT
   <213> Artificial Sequence
<220>
   <223> Synthetische Peptidsequenz
<400> 108
   Asn Tyr Gly Lys Tyr Ile
   1 5
<210> 109
   <211> 6
   <212> PRT
   <213> Artificial Sequence
<220>
   <223> Synthetische Peptidsequenz
<400> 109
   Asn Tyr Gly Lys Tyr Lys
   1 5
<210> 110
   <211> 6
   <212> PRT
   <213> Artificial Sequence
<220>
   <223> Synthetische Peptidsequenz
<400> 110
   Asn Tyr Gly Lys Tyr Leu
   1 5
<210> 111
   <211> 6
   <212> PRT
   <213> Artificial Sequence
<220>
   <223> Synthetische Peptidsequenz
<400> 111
   Asn Tyr Gly Lys Tyr Met
   1 5
<210> 112
   <211> 6
   <212> PRT
   <213> Artificial Sequence
<220>
   <223> Synthetische Peptidsequenz
<400> 112
   Asn Tyr Gly Lys Tyr Asn
   1 5
<210> 113
   <211> 6
   <212> PRT
   <213> Artificial Sequence
<220>
   <223> Synthetische Peptidsequenz
<400> 113
   Asn Tyr Gly Lys Tyr Pro
   1 5
<210> 114
   <211> 6
   <212> PRT
   <213> Artificial Sequence
<220>
   <223> Synthetische Peptidsequenz
<400> 114
   Asn Tyr Gly Lys Tyr Gln
   1 5
<210> 115
   <211> 6
   <212> PRT
   <213> Artificial Sequence
<220>
   <223> Synthetische Peptidsequenz
<400> 115
   Asn Tyr Gly Lys Tyr Arg
   1 5
<210> 116
   <211> 6
   <212> PRT
   <213> Artificial Sequence
<220>
   <223> Synthetische Peptidsequenz
<400> 116
   Asn Tyr Gly Lys Tyr Ser
   1 5
<210> 117
   <211> 6
   <212> PRT
   <213> Artificial
<400> 117
   Asn Tyr Gly Lys Tyr Thr
   1 5
<210> 118
   <211> 6
   <212> PRT
   <213> Artificial Sequence
<220>
   <223> Synthetische Peptidsequenz
<400> 118
   Asn Tyr Gly Lys Tyr Val
   1 5
<210> 119
   <211> 6
   <212> PRT
   <213> Artificial Sequence
<220>
   <223> Synthetische Peptidsequenz
<400> 119
   Asn Tyr Gly Lys Tyr Trp
   1 5
<210> 120
   <211> 6
   <212> PRT
   <213> Artificial Sequence
<220>
   <223> Synthetische Peptidsequenz
<400> 120
   Asn Tyr Gly Lys Tyr Tyr
   1 5
<210> 121
   <211> 6
   <212> PRT
   <213> Artificial Sequence
<220>
   <223> Synthetische Peptidsequenz
<400> 121
   Asn Tyr Gly Lys Tyr Glu
   1 5
<210> 122
   <211> 6
   <212> PRT
   <213> Artificial Sequence
<220>
   <223> Synthetische Peptidsequenz
<400> 122
   Asn Tyr Gly Lys Tyr Glu
   1 5
<210> 123
   <211> 6
   <212> PRT
   <213> Artificial Sequence
<220>
   <223> Synthetische Peptidsequenz
<400> 123
   Asn Tyr Gly Lys Tyr Glu
   1 5
<210> 124
   <211> 6
   <212> PRT
   <213> Artificial Sequence
<220>
   <223> Synthetische Peptidsequenz
<400> 124
   Asn Tyr Gly Lys Tyr Glu
   1               5
<210> 125
   <211> 6
   <212> PRT
   <213> Artificial Sequence
<220>
   <223> Synthetische Peptidsequenz
<400> 125
   Asn Tyr Gly Lys Tyr Glu
   1               5

## Beschreibung

### Hintergrund der Erfindung

Die vorliegende Erfindung betrifft ein Verfahren zur Herstellung eines Mikroarrays, einen Mikroarray zum Nachweis von Interaktionen zwischen Sondenmolekülen und Analytmolekülen aus einer Probe sowie die Verwendung des Mikroarrays für einen solchen Nachweis.

Im wissenschaftlichen Sprachgebrauch werden Sammlungen einer großen Anzahl unterschiedlicher Testverbindungen, die auf einer ebenen Fläche angeordnet sind, als "Arrays" bezeichnet. Die Testverbindungen werden häufig auch als Sonden bzw. Sondenmoleküle bezeichnet, die auf der ebenen Fläche gebunden bzw. immobilisiert sind. Diese Arrays erlauben das schnelle, gleichzeitige Testen aller Sondenmoleküle hinsichtlich ihrer Interaktion mit einem oder einer Mischung von Analyten in einer Probe. Die Analyten der Probe werden häufig als (Ziel-) moleküle bezeichnet. Der Vorteil eines planaren Arrays gegenüber einem Test (Assay) mit immobilisierten Sondenmolekülen auf beweglichen Elementen, wie beispielsweise Perlen (Beads), besteht darin, dass in einem Array die chemische Struktur und/oder die Identität der immobilisierten Sondenmoleküle über ihren Ort in der Arrayfläche genau definiert ist. Ein bestimmtes örtliches Testsignal, das beispielsweise durch eine Wechselwirkung zwischen dem Sondenmolekül und dem Analytmolekül entsteht, kann somit sofort unmittelbar einer Molekülart bzw. einem Sondenmolekül zugeordnet werden. Als Nachweis für eine Wechselwirkung zwischen Sondenmolekül und Analytmolekül kann auch die enzymatische Umsetzung der Sonde durch das Biomolekül verwendet werden, wodurch ein örtliches Testsignal auch verschwinden kann und somit dem direkten Nachweis dient. Insbesondere in miniaturisierter Form werden Arrays mit biologischen Sondenmolekülen auch als Biochips bezeichnet.

Beispiele für solche Arrays des Standes der Technik sind Nukleinsäurearrays aus DNA-Fragmenten, cDNAs, RNAs, PCR-Produkten, Plasmiden, Bakteriophagen und synthetischen PNA-Oligomeren, die mittels Hybridisierung unter Ausbildung eines Doppelstrangmoleküls zu komplementären Nukleinsäureanalyten ausgelesen werden. Daneben spielen Proteinarrays aus Antikörpern, aus in Zellen exprimierten Proteinen oder Phagen-Fusionsproteinen (Phage Display) eine wichtige Rolle. Daneben sind Verbindungsarrays aus synthetischen Peptiden, deren Analoga wie Peptoide, Oligo-Carbamate oder allgemein organisch chemischen Verbindungen bekannt, die beispielsweise mittels Bindung zu affinen Protein- oder anderen Analyten mittels enzymatischer Umsetzung ausgelesen werden. Daneben sind Arrays aus Chimären und Konjugaten der erwähnten Sondenmoleküle beschrieben worden.

Solche Arrays werden derzeit nach zwei verschiedenen Prinzipien durch Aufbringen der Sondenmoleküle auf zuvor speziell vorbereitete Materialoberflächen, beispielsweise Chip-Oberflächen, hergestellt. Eine Übersicht hierzu gibt Wölfl in: Transcript Laborwelt 3 (2000), 13-20.

Die beiden unterschiedlichen Prinzipien betreffen zum einen das einmalige Verteilen der Lösungen von vorgefertigten Sondenmolekülen auf der Oberfläche und/oder zum anderen das wiederholte, serielle Verteilen der Lösungen von Bausteinen für die parallele chemische Synthese der Sondenmoleküle in situ auf der Oberfläche.

Die Oberfläche mit den gebundenen Sondenmolekülen wird anschließend ganzflächig mit der Lösung der Analytmoleküle aus einer Probe in Kontakt gebracht, so dass bei erfolgter, spezifischer und selektiver Wechselwirkung zwischen dem Sondenmolekül und einem Analytmolekül ein Signal am Ort des Sondenmoleküls erzeugt wird. Dieses Signal kann entweder direkt entstehen, beispielsweise durch Bindung eines fluoreszenzmarkierten Biomoleküls, oder in weiteren Behandlungen mit Detektionsreagenzien beispielsweise als optisches oder radioaktives Signal erzeugt werden. Nach Auswaschen der Überschüsse an Analytmolekülen wird das Signal ausgelesen. Die vielfältigen technischen Details zur Durchführung und Detektion sind in Bowtell & Sambrook, DNA Microarrays: A cloning manual. Cold Spring Harbor Laboratory Press, Cold Spring Harbor, New York, (2003), ISBN 0-87969-624-9, beschrieben.

Die zunehmende Miniaturisierung der Arrays zu Mikroarrays besitzt vor allem bei der Analyse von biologischen Proben zum Beispiel in der medizinischen Diagnostik große Vorteile. So können mehr Sondenmoleküle pro Fläche angeordnet werden, die mehr Testsignale (Ergebnisse) mit der gleichen Menge der biologischen Probe liefern. Weil in bestimmten Fällen, wie beispielsweise bei humanen Biopsien, nur sehr wenig Ausgangsmaterial zur Verfügung steht, können erst durch eine solche Miniaturisierung diagnostische Analysen in einem breiten Umfang durchgeführt und die dahinterstehenden Fragen beantwortet werden.

Die Technologien zur Fertigung von Mikroarrays und Biochips mit Sondendichten von über 100 Sonden je cm² verwenden üblicherweise planare unporöse Glasträger als Oberfläche. Die Sonden werden hierbei als nahezu monomolekulare Schicht aufgebraucht (Southern et al., Nature Genetics Suppl. Nr. 1 (1999), 5-9; Xu et al., Molecular Diversity, (2004), 1-10). Die wesentlichsten Voraussetzungen für eine erfolgreiche, empfindliche Detektion der Analytmoleküle ist eine hohe Selektivität der Bindungsereignisse sowie eine hohe Komplexstabilität und Affinität zwischen dem Sondenmolekül und dem Analytmolekül. Für die letztere ist insbesondere eine geringe Dissoziationskonstante k_{off} entscheidend, die bestimmt, wie schnell der gebildete Komplex wieder zerfällt. Nur wenn die Dissoziationskonstante klein genug ist, bleibt das Analytmolekül ausreichend lange nach dem Einfangen am Ort des Sondenmoleküls, wenn die Überschüsse der Lösungen abgewaschen werden.

Solche vorteilhaften Bindungsverhältnisse lassen sich nahezu immer bei Nukleinsäuren erreichen, deren Detektion über Hybridisierung zu komplementären Nukleinsäuresträngen erfolgt. Bei genügender Länge der komplementären Stränge werden extrem hohe Stabilitäten als auch Selektivitäten erreicht. Diese vorteilhafte Situation ist eher selten für andere Sondenmoleküle, wie beispielsweise Peptide, Oligosaccharide oder chemische Verbindungen im Allgemeinen, und deren Komplexe mit Analytmolekülen, wie beispielsweise Proteinen. Die untersuchten analytischen Fragestellungen umfassen eine große Spannbreite von Komplexstabilitäten, mit Dissoziationskonstanten von pM, wie beispielsweise Streptavidin mit Biotin bis zu mM für Protein/Wirkstoff Interaktionen.

Für Mikroarrayanalysen mit Komplexen geringer Stabilität wurde die Erhöhung der lokalen Konzentration der Sondenmoleküle vorgeschlagen. Dadurch können beim Waschvorgang dissoziierte Zielmoleküle schneller wieder mit anderen Sondenmolekülen in räumlicher Nähe zurückbinden ("re-binding"). Eine signifikante Erhöhung der lokalen Sondenkonzentration wird aber erst erreicht, wenn statt einer 2D planaren molekularen Schicht eine 3D Schicht verwendet wird. Beispiele hierfür sind die Verwendung poröser Schichten, bei denen auch die innere Oberfläche nutzbar ist.

Beispiele aus der Praxis hierfür sind die SPOT Synthese auf Membranen aus Cellulose, Polypropylen oder Teflon (The SPOT-Synthesis Technique: Synthetic Peptide Arrays on Membrane Supports. In: Methods of parallel peptide synthesis and their contributions to deciphering molecular interactions in the immune system. Guest Editor: C. Granier, Part 3, The SPOT method of peptide synthesis: the role of arrayed peptides in revealing key features of antigen-antibody recognition. Special Issue of the Journal of Immunological Methods; Frank, J. Immunol. Meth. 267 (2002), 13-26). Protein-Peptidkomplexe auf solchen Membranträgern können noch bis zu einer Dissoziationskonstante von nahezu 1 mM nachgewiesen werden (Hoffmüller et al., Angew. Chem. Int. Ed. 38 (1999) 2000-2003).

Weitere Beispiele sind die so-genannten Patcharrays mit kleinsten Kissen oder Erhebungen von Polyacrylamid, das kovalent an Glas gebunden vorliegt (Yershov et al., PNAS 93 (1996), 4913-4918). Alternativ dazu können auch kleine, räumlich getrennte, pfropfartige Polymerisierungen auf Polypropylen verwendet werden (DE 103 40 429). Ferner sind Pixelarrays mit kleinsten Erhebungen aus Polyacrylsäure-Graft auf Polypropylen beschrieben worden (WO 02/066984).

Bei der Firma Schleicher & Schüll, Deutschland sind darüber hinaus Nitrocellulose- oder Nylonschichten, die auf Glasträgern gebunden sind, kommerziell erhältlich, wobei die empfohlene Auftrags- bzw. Spotdichte bei 100 Spots pro cm² liegt. Dies ist durch die hohe Saugkraft der Schicht und infolge von Diffusion der aufgebrachten Sondenmoleküle nicht viel weiter miniaturisierbar.

Ferner sind Flächenmaterialien mit parallel ausgerichteten Poren, die auch als Flow-Through-Chips bezeichnet werden, beschrieben worden (Benoit et al., Anal. Chem. 73 (2001), 2412-2420).

Darüber hinaus wurde beschrieben, dass Sondenmoleküle zunächst an einen löslichen polymeren Träger wie beispielsweise Proteine oder Polysaccharide gekoppelt werden können, wobei dieses lösliche Konjugat anschließend mit einem Mikrodosierer auf beispielsweise Glas- oder Kunststoffoberflächen gespottet wird. Das eingetrocknete Konjugat wird bei entsprechender Wahl des Trägers ein poröses Präzipitat bilden, das aber fest auf der Chipoberfläche haftet. Damit wird eine lokale Situation ähnlich einer porösen Membranschicht geschaffen (Xu et al., Molecular Diversity, (2004), 1-10, zur Veröffentlichung eingereicht). Für dieses Verfahren müssen die Sondenmoleküle jedoch in einer Form vorliegen oder hergestellt werden, die eine gezielte Verknüpfung mit dem Trägermaterial erlaubt. Die Autoren schlagen hierzu die chemische Ligation zwischen einem speziellen, ketonmodifizierten polymeren Träger und amino-oxylacetylmodifizierten Sonden vor. Nachteilig ist jedoch insbesondere, dass beide Komponenten speziell hergestellt und erst in einem anschließenden Verknüpfungsschritt zusammengebracht werden. Dies muss darüber hinaus sicher und zuverlässig für viele tausend Sonden durchgeführt werden.

Bei allen im Stand der Technik beschriebenen Verfahren zur Herstellung von Mikroarrays sind insbesondere Sondenmoleküle mit niedrigem Molekulargewicht wie beispielsweise Peptide oder kleine organische Moleküle problematisch. Diese müssen aufwändig durch kombinatorische oder parallele chemische Synthese erzeugt werden, welche überwiegend auf der Oberfläche eines polymeren Trägers durchgeführt wird. Einen Überblick über die chemische Festphasensynthese nach Merrifield und seine vielfältigen Abwandlungen, insbesondere für die kombinatorische und parallele Herstellung von Verbindungsbibliotheken ist von Dörwald, Organic Synthesis on Solid Phase, (2000) Wiley-VCH Verlag GmbH, Weinheim, Deutschland, ISBN 3-527-29950-5, beschrieben.

Der vorliegenden Erfindung liegt daher die Aufgabe zugrunde, ein neues Verfahren zur Herstellung von Mikroarrays für chemisch-synthetische Sondenmoleküle zu schaffen, das einfacher durchzuführen ist, alle Vorteile der Festphasensynthese einschließlich der automatisierten Durchführung nutzen kann und darüber hinaus für Sondenmoleküle mit niedrigem Molekulargewicht geeignet ist.

### Zusammenfassung der Erfindung

Diese Aufgabe wird gelöst durch ein Verfahren zur Herstellung eines Mikroarrays, wobei das Verfahren die Schritte umfasst:
(a) Zwei- bis mehrstufiges Synthetisieren von Sondenmolekülen an einem polymeren Träger, wobei Bindungen zwischen den Sondenmolekülen und dem polymeren Träger ausgebildet werden;
(b) Auflösen des polymeren Trägers mit den synthetischen Sondenmolekülen gemäß Schritt (a), wobei die Bindungen zwischen dem polymeren Träger und den Sondenmolekülen erhalten bleiben;
(c) Gegebenenfalls Reinigen der an den polymeren Träger gebundenen Sondenmoleküle;
(d) Lösen des polymeren Trägers mit den gebundenen Sondenmolekülen; sowie
(e) Aufbringen der Lösung enthaltend den polymeren Träger mit den gebundenen Sondenmolekülen als Mikrotropfen auf eine planare Oberfläche.

Das erfindungsgemäße Verfahren vereint daher eine Reihe von Arbeitsschritten des Standes der Technik, insbesondere vereint es die chemische Synthese und die Konjugation, beispielsweise gemäß Xu et al., in Molecular Diversity, (2004), 1-10, durch die Verwendung desselben polymeren Trägermaterials für beide Schritte. Dadurch wird eine erhebliche Vereinfachung des Standes der Technik erreicht.

Ferner wird die Aufgabe gelöst durch einen Mikroarray gemäß Ansprüch 14, der nach dem erfindungsgemäßen Verfahren hergestellt worden ist.

Das erfindungsgemäße Verfahren nutzt parallel und kombinatorische Syntheseverfahren zur Herstellung von Sondenmolekülen und Molekülbibliotheken. Durch die Wahl eines geeigneten polymeren Trägers können Mikroarrays direkt für die Interaktionsanalyse hergestellt werden, deren Qualität und auch Sensitivität von bisher beschriebenen Makro-Testverfahren gleichkommt. Für dieses Verfahren können alle chemischen Verbindungen, die bei Makro-Testverfahren verwendet werden, ebenfalls eingesetzt werden.

Der besondere Vorteil des erfindungsgemäßen Verfahrens liegt darin, dass aus einem sehr kleinen Synthesesatz eine große Anzahl gleicher Kopien von Mikroarrays hergestellt bzw. gedruckt werden können. Aufgrund dessen dass dieselbe Lösung für jede Kopie verwendet wird, haben die Arraykopien eine sehr vergleichbare Qualität. Dies ist ein signifikanter Unterschied zu beispielsweise durch in situ Synthese hergestellten Arrays, bei denen in jeder Kopie die Sondenmoleküle separat synthetisiert werden müssen. Legt man die im Beispielteil beschriebenen sowie die in den speziellen Ausführungsformen der Erfindung bevorzugten Werte für das Auflösen des ausgefällten polymeren Trägers zugrunde, so können aus einer Synthese von ca. 50 nmol je Sonde auf den Spots einer Cellulosemembran bis zu 10 Millionen Arrays mit der jeweils gleichen Qualität hergestellt werden.

Gegenüber den bisher beschriebenen Mikroarrays aus synthetischen Sondenmolekülen, die durch Reimmobilisierung der Sondenmoleküle nach der Synthese hergestellt werden (Xu et al., (2004)), weist das erfindungsgemäße Verfahren einen weiteren Vorteil für die Herstellung insbesondere von Mikroarrays aus kleinen organischen Molekülen für die pharmazeutische Wirkstoffsuche auf. Neben der chemischen Funktion des Sondenmoleküls zur Verankerung auf dem Syntheseträger wird keine zusätzliche chemische Funktion im Molekül für die Konjugation benötigt, was den Spielraum der Diversität kleiner organischer Moleküle deutlich vergrößert.

### Figurenbeschreibung

Figur 1 zeigt die Ergebnisse der Synthese und einen Antikörperbindungstest einer Kollektion von 125 Peptidsonden nach dem SPOT-Syntheseverfahren auf einer Cellulosemembran mit 5 mm Spot-Abstand.
Figur 1a zeigt ein Abbild der MTT/BCIP-Testsignale;
Figur 1b zeigt die quantitative Auswertung der Testsignale und deren Darstellung als 2D-Karte.
Figur 2 zeigt einen Antikörperbindungstest derselben Sondenpeptide wie in Figur 1 dargestellt, die nach dem SPOT-Syntheseverfahren auf einer auflösbaren Cellulosemembran hergestellt wurden, nach Ausstanzen, Auflösen und Spotten auf einen unbehandelten Glasmikroskopträger, wobei der SPOT-Abstand 0,5 mm beträgt.
Figur 2a zeigt ein Abbild der Cy5 Fluoreszenztestsignale;
Figur 2b zeigt die quantitative Auswertung der Testsignale und die Darstellung als eine 2D-Karte.

### Ausführliche Beschreibung der Erfindung

Für das erfindungsgemäße Verfahren werden die Sondenmoleküle durch kombinatorische oder parallele chemische Synthese an einem geeigneten polymeren Träger hergestellt und verbleiben an diesem immobilisiert durch die Wahl einer stabilen, chemischen Verbindung, die insbesondere als stabiler chemischer Anker ausgebildet sein kann, zum Beispiel in Frank und Overwin, In: Methods in Molecular Biology, 66: Epitope Mapping Protocols (G. E. Morris, Ed.), The Humana Press Inc., Totowa, USA (1996), 149-169). Eine Abspaltung aller Schutzgruppen ist möglich und eine Vorreinigung der Sondenmoleküle durch Waschen ist empfehlenswert. Auf diese Weise sind alle Vorteile der Festphasensynthese einschließlich der automatisierten Durchführung nutzbar. Wahlweise kann ein geringer Teil der Sondenmoleküle vom Trägermaterial abgespalten werden, um die Synthesequalität mit Hilfe analytischer Techniken zu überprüfen. Für eine solche Abspaltung muss ein geeigneter Linker vorgesehen sein; vgl. zum Beispiel Frank und Overwen, *loc*. *cit*. Anschließend wird das mit den Sondenmolekülen beladene Trägermaterial durch eine spezielle chemische Behandlung in einem Lösungsmittel aufgelöst, die ggf. durch einen teilweisen Abbau des Polymers unterstützt werden kann. Die Sondenmoleküle selbst als auch die chemische Verknüpfung der Sondenmoleküle mit dem Trägermaterial bleiben bei diesen Schritten jedoch erhalten. Anschließend können die so erhaltenen Lösungen der an das Trägermaterial gebundenen Sondenmoleküle wahlweise verdünnt werden und mit einem Mikrodosierer auf beispielsweise Glas- oder Kunststoffoberflächen gespottet werden. Die Mikrotropfen werden auf der Oberfläche eingetrocknet. Die so erhaltenen Mikroarrays mit immobilisierten Sondenmolekülen werden anschließend für die Interaktionsanalyse von Analytmolekülen eingesetzt.

Das polymere Trägermaterial, das in dem erfindungsgemäßen Verfahren eingesetzt wird, sollte unlöslich in den meisten organischen Lösungsmitteln sein, die für die chemische Synthese eingesetzt werden. Ferner sollte es gegenüber den meisten chemischen Synthesereaktionen, die für den chemischen Aufbau der Sondenmoleküle verwendet werden, inert sein. Eine genügend hohe Beladung mit chemischen Funktionalitäten für die Einführung an Anker/Linker-Verbindungen, die zum Aufbau der chemischen Sonden gebraucht werden, ist außerdem von Vorteil. Ferner sollte das polymere Trägermaterial durch ein für das Sondenmolekül nicht destruktives Verfahren in einem Lösungsmittel lösbar gemacht werden können, das sich für das Mikrospotten eignet. Ferner sollte das polymere Trägermaterial die Ausbildung eines auf Glas- oder Kunststoffoberflächen gut haftbaren Präzipitats erlauben. Ferner ist die Ausbildung eines unter den Bedingungen der Interaktionsanalyse nicht auflösbaren Präzipitats von entscheidendem Vorteil bzw. unverzichtbar. Darüber hinaus hat sich die Ausbildung eines porösen Präzipitats, das einen genügend guten Zugang der Ziel- bzw. Analytmoleküle in den Proben zu den immobilisierten Sondenmolekülen gewährleistet, als besonders vorteilhaft erwiesen.

Es hat sich insbesondere als vorteilhaft erwiesen, wenn die Bindungen zwischen den Sondenmolekülen und dem polymeren Träger chemische Bindungen, insbesondere kovalente chemische Bindungen sind. Kovalente Bindungen sind sehr feste chemische Bindungen, welche eine dauerhafte, stabile Verbindung zwischen den Sondenmolekülen und dem polymeren Träger selbst während des Auflösens des polymeren Trägers erlauben.

Der polymere Träger ist vorzugsweise aus einem porösen Material ausgebildet. Papier, insbesondere Cellulosepapier, ist besonders geeignet; vgl. zum Beispiel Frank, Nucleic Acids Res. 11 (1983), 4365-4377; Frank und Döring, Tetrahedron 44, 19 (1988), 6031-6040; Frank, Tetrahedron 48 (1992), 9217-9232; Dittrich et al., Bioorg. Med. Chem. Lett. 8 (1998), 2351-2356.

Das Auflösen des polymeren Trägers geschieht beispielsweise in einer Mischung aus Trifluoressigsäure (TFA), Dichlormethan, Tri-isobutylsilan (TIBS) und Wasser. Gleichzeitig können mit dieser Lösung die noch vorhandenen Seitenkettenschutzgruppen an den Sondenmolekülen, die beispielsweise Peptidsonden sein können, abgespalten, so dass nur noch das entschützte Sondenmolekül bzw. Peptid gebunden am aufgelösten polymeren Trägermaterial vorliegt.

In einer besonders bevorzugten Ausführungsform der Erfindung weist die Mischung zur Auflösung des polymeren Trägers ca. 60-100 Vol.-%, und insbesondere 80-90 Vol.-% Trifluoressigsäure, vorzugsweise ca. 85 Vol.-%, ca. 0-15 Vol.-% und insbesondere 5-15 Vol.-% Dichlormethan, vorzugsweise ca. 7 Vol.-%, ca. 0-5 Vol.-% und insbesondere 2-5 Vol.-% Tri-isobutylsilan, vorzugsweise ca. 3 Vol.-% und ca. 0-5 Vol.-% und insbesondere 2-5 Vol.-% Wasser, vorzugsweise ca. 5 Vol.-%, auf.

Es hat sich als vorteilhaft erwiesen, wenn die Reaktionszeit zum Auflösen des polymeren Trägers ca. 0,1 - 24 Stunden und insbesondere 2 - 24 Stunden, vorzugsweise ca. 16 Stunden beträgt.

Das Waschen des polymeren Trägers mit den gebundenen Sondenmolekülen kann bevorzugt in Diethylether, Aceton oder Tert-butylmethylether erfolgen. Diethylether ist besonders bevorzugt. Insgesamt sind alle Fällungsmittel geeignet, in denen sich der polymere Träger nicht löst. Dagegen sollten die Verunreinigungen und abgespaltenen Schutzgruppen in dem Fällungsmittel/Lösungsmittel gut lösen. Drei- bis fünfmaliges Waschen hat sich bewährt, wobei jedoch dreimaliges Waschen in der Regel genügt.

Das Lösen des polymeren Trägermaterials mit den daran immobilisierten Sondenmolekülen erfolgt beispielsweise in Wasser, DMF, NMP oder DMSO, wobei DMSO bevorzugt ist. Alle Lösungsmittel sind geeignet, in denen sich der polymere Träger und die Sondenmoleküle gut lösen. Darüber hinaus muss das Lösungsmittel für den nachfolgenden Schritt des Mikrospottens geeignet sein. Für DMSO wurde herausgefunden, dass es die größte Lösungsvermittlung besitzt und sich trotz seines sehr hohen Siedepunktes gut zum Mikrospotten eignet. Die verwendete Menge an Lösungsmittel ist insbesondere abhängig vom verwendeten polymeren Träger. Der Verdünnungsfaktor beträgt 1:5 bis 1:100, vorzugsweise 1:20. Der Verdünnungsfaktor ist abhängig vom verwendeten Polymerträger und bestimmt die gewünschte Spotmorphologie auf der planaren Oberfläche.

In einer bevorzugten Ausführungsform sind die Sondenmoleküle kurzkettige Peptide, Polypeptide, Polysaccharide oder Nukleinsäuren, DNA- oder RNA-Moleküle und insbesondere kleine organische Moleküle.

Erfindungsgemäß fällt ein Mikrochip, insbesondere eines Biochip an, wenn auf eine planare Oberfläche die Lösung enthaltend das polymere Trägermaterial mit den immobilisierten Sondenmolekülen aufgespottet wird.

Weil das erfindungsgemäße Verfahren keine chemische oder physikalische Vorbehandlung der planaren Oberfläche erfordert, kann jedes denkbare Material als Oberfläche benutzt werden. Als insbesondere vorteilhaft hat sich Metall, Glas, Plastik oder Keramik erwiesen, insbesondere ein Mikroskopglasträger. Natürlich sind auch andere Oberflächen denkbar.

Alternativ dazu ist es auch möglich, als polymeren Träger ein Protein zu verwenden, an das Sondenmoleküle synthetisiert werden (Hansen et al., Int. J. Peptide Protein Res. 41 (1993), 237-245). Die Sondenmoleküle werden hierbei parallel in getrennten Reaktionsgefäßen synthetisiert und das Trägermaterial nach jedem Syntheseschritt durch Ausfällen und Zentrifugation aus dem Rektionsgemisch abgetrennt. Die weiteren Schritte der Sondensynthese und Mikroarrayherstellung werden wie bei der Verwendung von Cellulose als polymerer Träger durchgeführt.

Als weiteres mögliches Trägermaterial kommt neben Cellulose oder Proteinen beispielsweise auch Disulfid-vernetztes Polyacrylat in Betracht (Goddard et al., J.Chem. Soc. Chem. Commun. (1988), 1025-1027).

Die vorliegende Erfindung betrifft darüber hinaus einen Mikroarray gemäß Ansprüch 14, wobei der Mikroarray durch das erfindungsgemäße Verfahren hergestellt worden ist.

In diesem Mikroarray sind die Sondenmoleküle vorzugsweise kurzkettige Peptide, Polypeptide, Polysaccharide oder Nukleinsäuren, DNA- oder RNA-Moleküle, insbesondere kleine organische Moleküle.

Eine Probe im Sinne des Verfahrens umfasst jede Art Lösung von Analytmolekülen, die eine Interaktion oder chemische bzw. enzymatische Reaktion mit den Sondenmolekülen auf dem Array eingehen kann. Hierzu gehören insbesondere biologische Proben, die gewonnen werden durch die Entnahme von biologischen Flüssigkeiten wie Blut, Serum, Sekret, Lymphe, Dialysat, Liquor, Pflanzensaft, Körpersaft aus Insekten, Würmern, Maden, usw. Ferner ist die Extraktion aus natürlichen Quellen wie Biopsien, tierischen und pflanzlichen Organen oder Teilen, Zell-Insekten-, Wurm-, Bakterien-, Mikroben-, und Parasitenmasse sowie überstände von Zellkulturen und von Bakterien-, Mikroben- oder Parasitenkulturen umfasst. Eine Probe kann auch eine chemisch-synthetische Probe enthaltend beispielsweise Oligonukleotide, PNAs, RNAs, Peptide und synthetische Proteine, organisch synthetische Rezeptoren, Reagenzien und/oder Käfigmoleküle, sein.

Insbesondere kann als Probe eine menschliche Probe und vorzugsweise eine Probe aus einer menschlichen Biopsie verwendet werden.

Ferner betrifft die Erfindung die Verwendung des nach dem erfindungsgemäßen Verfahren herstellbaren Mikroarray zum Nachweis von Interaktionen zwischen Sondenmolekülen und Analytmolekülen aus einer Probe.

Im Folgenden wird das erfindungsgemäße Verfahren am Beispiel synthetischer Peptide als Sondenmoleküle beschrieben. Es wird deren chemische Synthese auf Cellulosepapier als polymeren Träger nach dem Filterverfahren, das Spotverfahren oder das Cut & Combine-Verfahren, die Auflösung der polymeren Trägersegmente mit den immobilisierten Sonden mittels Trifluoressigsäure (TFA), die Reinigung der immobilisierten Sonden durch Fällung in Ether sowie die Lösung des Präzipitats in Dimethylsulfoxid (DMSO) sowie das Spotten der DMSO-Lösungen auf Glasträger beschrieben. Nach dem Eintrocknen und der Inkubation der gespotteten Glasarrays mit der Lösung eines Antikörpers können an einzelne Sondenmoleküle gebundene Antikörper mit einem fluoreszenzmarkierten, sekundären Antikörper nach dem ELI-SA Prinzip nachgewiesen werden.

Gängige molekularbiologische Arbeitsmethoden sind hier nicht im Detail beschrieben, sie können aber nachgelesen werden bei Bowtell und Sambrook, In: DNA Microarrays: A cloning manual. Cold Spring Harbor Laboratory Press, Cold Spring Harbor, New York, (2003), ISBN 0-87969-624-9; Frank und Overwin, In: Methods in Molecular Biology, 66: Epitope Mapping Protocols (G. E. Morris, Ed.), The Humana Press Inc., Totowa, USA (1996), 149-169; Harlow und Lane, In: Antibodies: A laboratory manual. Cold Spring Harbor, New York, (1988); und Sambrook et al., Molecular Cloning: A laboratory manual. Cold Spring Harbor, New York (1989), ISBN 0-87969-309-6.

### Beispiel

### Schritt 1: Synthese der Sondenmoleküle am polymeren Träger

120 Peptide wurden nach der SPOP-Methode (Frank, 1992) synthetisiert. Ausgehend von der Sequenz Ac-NYGKYE- des Epitoppeptids für den monoklonalen Anti-TTL-Antikörper 1D3 stellen diese 120 Peptide einen kompletten Substitutionssatz dar. Der monoklonale Anti-TTL-Antikörper 1D3 ist beschrieben bei Erck et al., Neurochem. Res. 25 (2000), 5-10. Jede Aminosäure der Sequenz wurde durch jede andere der 20 proteinogenen Aminosäuren ersetzt. Die Liste der 120 synthetisierten Peptide ist weiter unten gezeigt.

Ein Blatt Cellulosemembran (Whatman 540) wurde mit β-Alanin verestert. Das Verfahren hierfür ist beschrieben in Frank und Overwin, In: Methods in Molecular Biology, 66: Epitope Mapping Protocols (G. E. Morris, Ed.), The Humana Press Inc., Totowa, USA (1996), 149-169. Die erhaltene Beladung nach β-Alaninveresterung betrug 1,05 µmol/cm². Die nachfolgend durchgeführte schrittweise Peptidsynthese zum Aufbau der Sondenmoleküle folgte dem Verfahren, das bei Frank und Overwin (siehe oben) beschrieben ist. Für die Synthese wurde der vollautomatische Syntheseroboter MultiPep der Firma Intavis Bioanalytische Instrumente AG in Köln, Deutschland nach den Angaben des Herstellers verwendet.

Nach Kupplung der letzten Aminosäure wurde die N-terminale Fmoc-Schutzgrupe mit einer 20 %igen Piperidinlösung in Dimethylformamid (DMF) abgespalten. Anschließend wurden die freien Aminogruppen mit Bromphenolblau angefärbt. Die Blockierung der freien N-terminalen Aminofunktionen erfolgte mit einer 2 %igen Essigsäureanhydridlösung in DMF, bis die blaue Färbung verschwunden war. Anschließend wurde die Membran insgesamt 3 x 10 min mit 4 x DMF und für jeweils 3 min 3 x mit Ethanol gewaschen. Die Membran wurde anschließend an der Luft getrocknet. Danach wurden die einzelnen Spots ausgestanzt und in die Vertiefungen (Näpfe) von zwei 96er Deepwell-Mikrotiterplatten (2 ml je Vertiefung) aus Polypropylen gegeben. Der Durchmesser der ausgestanzten Spots betrug 3,5 mm.

### Schritt 2: Auflösen des polymeren Trägers unter Erhalt der kovalenten Bindung zwischen dem Träger und dem Sondenmolekül

Auf jeden einzelnen Spot (ca. 0,1 cm², 50 nmol Sonde) wurden 300 µl einer TFA Lösung gegeben. Die Deepwell-Mikrotriterplatten wurden verschlossen und für 10 min mit Ultraschall behandelt. Danach wurden sie für 1 h geschüttelt. Nach einer zweiten 10 min Ultraschallbehandlung wurden die Deepwell-Mikrotiterplatten über Nacht bei Raumtemperatur geschüttelt, bis sich alle diese Spots vollständig aufgelöst hatten. Gleichzeitig wurden mit dieser Lösung die noch vorhandenen Seitenkettenschutzgruppen an den Peptidsonden abgespalten, so dass nur noch entschütztes Peptid am aufgelösten polymeren Trägermaterial vorlag. Die TFA Lösung enthielt 85 Vol.-% Trifluoressigsäure, 7 Vol.-% Dichlormethan, 3 Vol.-% Tri-isobutylsilan und 5 Vol.-% Wasser. Die gesamte Reaktionszeit über Nacht betrug ca. 16 Stunden.

### Schritt 3: Ausfällen und Reinigen der synthetisierten Verbindung

In jede einzelne Vertiefung der 96er Deepwell-Mikrotiterplatte, welche die in der TFA Lösung gelösten Spots enthielten, wurden jeweils 1 ml Diethylether zum Ausfällen des gelösten polymeren Trägers gegeben. Anschließend wurden die Mikrotiterplatten für 10 min geschüttelt und darauffolgend für 15 min bei 4°C im Kühlschrank stehen gelassen. Anschließend wurde für 6 min bei 3000 Umdrehungen/min zentrifugiert. Der Überstand, der unter anderem die abgespaltenen Schutzgruppen enthielt, wurde vorsichtig abpipettiert. Als Rückstand verblieb der polymere Träger mit den daran befindlichen Sondenmolekülen in den Vertiefungen der Mikrotiterplatten zurück. Der Rückstand wurde dreimal mit Ether gewaschen. Hierfür wurde zunächst in jede Vertiefung je 1 ml Ether zu dem Präzipitat gegeben, die Mikrotiterplatten für 10 min geschüttelt, anschließend für 10 min bei 3000 Umdrehungen/min zentrifugiert und die überstehende Lösung abpipettiert. Nach dem letzten Waschvorgang wurden die Mikrotiterplatten offen stehengelassen, um Reste des Ethers abzudampfen.

### Schritt 4: Analytische Qualitätskontrolle der Sondenmoleküle

Von der in Schritt 2 erhaltenen Lösung mit TFA wurde ein Teil, der 5 nmol Sonde entsprach, abgenommen und separat wie in Schritt 2 und 3 beschrieben, behandelt. Der ausgefällte und getrocknete Rückstand wurde in einem fest verschlossenen Eppendorf Röhrchen mit 20 µl einer 33 %igen wässrigen Ammoniaklösung bei 80°C für 4 h behandelt. Dadurch wurde die Esterbindung der Sondencellulose zum Amid gespalten, und die Sonde wurde von dem Celluloseträger abgelöst. Anschließend wurden 0,5 µl dieser Lösung mit 0,5 µl α-Zimtsäure gemischt und auf einen MALDI-Träger aufgetragen. Die Sonde und die Synthesenebenprodukte wurden über die Messung des Molgewichts mittels Matrix-Assisted-Laser-Desorption Ionisation (MALDI) Massenspektroskopie identifiziert und damit die Identität sowie die Reinheit des Syntheseproduktes bestimmt.

### Schritt 5: Lösen des polymeren Trägers

In jede einzelne Vertiefung der Polypropylen-Deepwell-Mikrotiterplatte mit den getrockneten Rückständen aus Schritt 3 wurden 500 µl DMSO gegeben und für 10 min mit Ultraschall behandelt. Danach wurde die Platte über Nacht geschüttelt, bis sich der ausgefallene polymere Träger mit dem daran befindlichen Sondenmolekül vollständig aufgelöst hatte. Geringe Reste des Präzipitats wurden durch Zentrifugieren für 10 min bei 3000 Umdrehungen/min abgetrennt. Von diesen so erhaltenen Lösungen wurde 3 µl abgenommen und in einer 96er Standard-Mikrotiterplatte mit jeweils 57 µl DMSO verdünnt, so dass eine 1:20 Verdünnung erhalten wurde. Diese Lösungen wurden anschließend für das Spotten verwendet.

### Schritt 6: Herstellen des Mikroarrays durch Spotten der Lösungen auf planaren Oberflächen

Die 1-20 nl großen Tropfen der 1:20 Verdünnungen wurden mit einem Mikrodosierer (MicroGrid II der Firma BioRobotics; z.B. Gilson Model 231; GeSiM Nano-Plotter) auf eine planare Oberfläche aufgetragen. Es wurden Glasobjektträger Superfrost der Firma Menzel, Braunschweig, Deutschland in der Größe 76 x 26 mm, geschnitten verwendet. Es wurde ein Raster von 25 x 5 Punkten gespottet, was 125 Spots entsprach. Aufgrund der Tatsache, dass nur 120 Peptide vorhanden waren, wurden die letzten 5 Spots im Array zur Kontrolle mit der Originalsequenz Ac-NYGKYE gespottet. Auf jeden Glasobjektträger wurden 9 Replikate des 25 x 5 Arrays gespottet, wobei ein 64 pin-Tool mit 1 Split Pin (etwa 1 nl Transfer) zum Spotten benutzt wurde. Der Abstand der Spots untereinander betrug 0,5 mm. Die gespotteten Glasträger wurden für 2 h bei 50°C getrocknet und anschließend bei 4°C belagert.

### Schritt 7: Antikörpertest

Der Bindungstest mit dem 1D3 Antikörper wurde entsprechend dem bei Frank und Overwin, 1996 (siehe oben) beschriebenen Verfahren durchgeführt. Die Inkubation mit dem 1D3 Antikörper und einem zweiten Nachweisantikörper erfolgte durch Auftropfen der Antikörperlösung (60 µl Lösung mit 10µg/ml 1D3 Antikörper) auf dem Glasobjektträger und anschließendem Abdecken mit einem Glas-Deckgläschen. Als Nachweisantikörper diente der bei Frank und Overwin, 1996 beschriebene Ziege-anti-Maus-Antikörper, der jedoch mit dem Fluoreszenzfarbstoff Cy5 markiert war. Die Markierung erfolgte nach den Angaben des Herstellers (Amersham Biosciences, Produktnummer PA25001). Dadurch war eine Auswertung des Mikroarraytests mit einem Fluoreszenzdetektor möglich. Das Auslesen der Fluoreszenzsignale erfolgte mit einem ArrayWorx-Scanner der Firma Applied Precision mit den vom Hersteller für die Cy5 Markierung voreingestellten Wellenlängenbereichen für Anregungs- und Emissionswellenlänge.

Alternativ dazu kann als Nachweisantikörper auch das bei Frank und Overwin, 1996 aufgeführte Konjugat mit alkalischer Phosphatase verwendet werden, so dass ein Nachweis durch Anfärben mit MTT und BCIP möglich ist. Die Auswertung erfolgt dann wie herkömmlich mit einem konventionellen Scanner.

### Liste der verwendeten Abkürzungen

- DMF: Dimethylformamid
- NMP: N-Methylpyrrolidon
- DMSO: Dimethylsulfoxid
- EtOH: Ethanol
- TFA: Trifluoressigsäure
- TIBS: Tri-isobutylsilan
- DCM: Dichlormethan
- MTT: Methyltiazolyldiphenyl-tetrazolium-bromid
- BCIP: Brom-chlor-indolyl-phosphat

### Liste der synthetisierten Peptidsequenzen

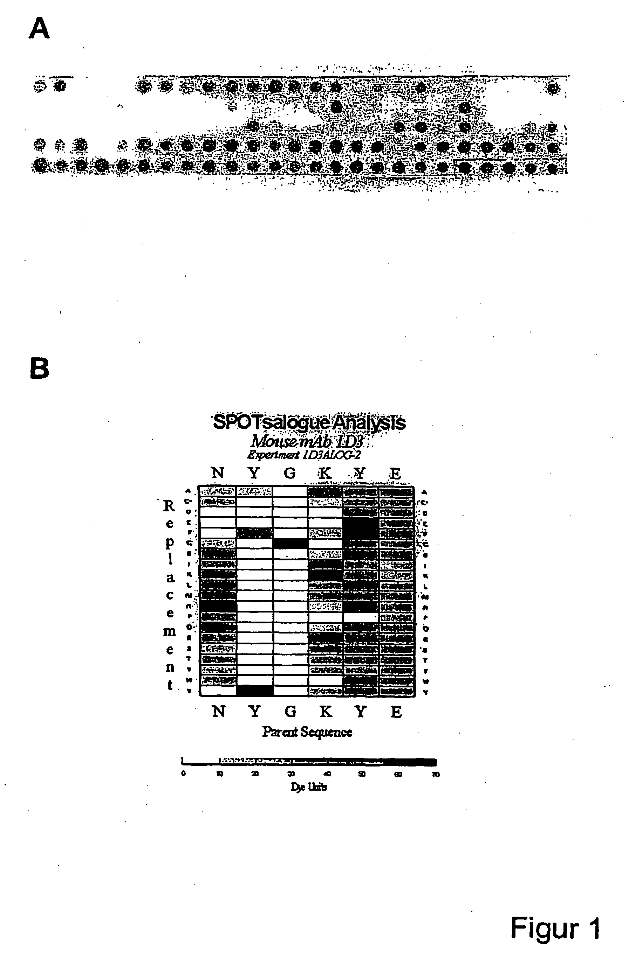

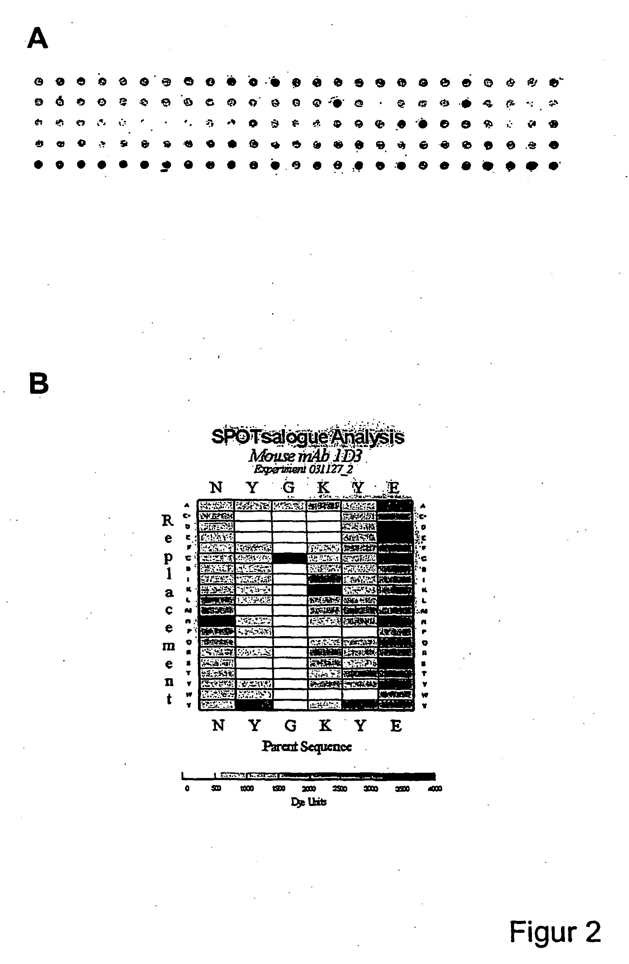

Die Angabe der Position bezieht sich auf die Position der Peptide im Array auf dem planaren Träger/Glasslide, wie es in Figur 1A und 2A zu sehen ist.

| **Position** | **Peptidsequenz** | **SEQ ID NO:** |
|---|---|---|
| A1 | AYGKYE | SEQ ID NO: 1 |
| A2 | CYGKYE | SEQ ID NO: 2 |
| A3 | DYGKYE | SEQ ID NO: 3 |
| A4 | EYGKYE | SEQ ID NO: 4 |
| A5 | FYGKYE | SEQ ID NO: 5 |
| A6 | GYGKYE | SEQ ID NO: 6 |
| A7 | HYGKYE | SEQ ID NO: 7 |
| A8 | IYGKYE | SEQ ID NO: 8 |
| A9 | KYGKYE | SEQ ID NO: 9 |
| A10 | LYGKYE | SEQ ID NO: 10 |
| A11 | MYGKYE | SEQ ID NO: 11 |
| A12 | NYGKYE | SEQ ID NO: 12 |
| A13 | PYGKYE | SEQ ID NO: 13 |
| A14 | QYGKYE | SEQ ID NO: 14 |
| A15 | RYGKYE | SEQ ID NO: 15 |
| A16 | SYGKYE | SEQ ID NO: 16 |
| A17 | TYGKYE | SEQ ID NO: 17 |
| A18 | VYGKYE | SEQ ID NO: 18 |
| A19 | WYGKYE | SEQ ID NO: 19 |
| A20 | YYGKYE | SEQ ID NO: 20 |
| A21 | NAGKYE | SEQ ID NO: 21 |
| A22 | NCGKYE | SEQ ID NO: 22 |
| A23 | NDGKYE | SEQ ID NO: 23 |
| A24 | NEGKYE | SEQ ID NO: 24 |
| A25 | NFGKYE | SEQ ID NO: 25 |
| B1 | NGGKYE | SEQ ID NO: 26 |
| B2 | NHGKYE | SEQ ID NO: 27 |
| B3 | NIGKYE | SEQ ID NO: 28 |
| B4 | NKGKYE | SEQ ID NO: 29 |
| B5 | NLGKYE | SEQ ID NO: 30 |
| B6 | NMGKYE | SEQ ID NO: 31 |
| B7 | NNGKYE | SEQ ID NO: 32 |
| B8 | NPGKYE | SEQ ID NO: 33 |
| B9 | NQGKYE | SEQ ID NO: 34 |
| B10 | NRGKYE | SEQ ID NO: 35 |
| B11 | NSGKYE | SEQ ID NO: 36 |
| B12 | NTGKYE | SEQ ID NO: 37 |
| B13 | NVGKYE | SEQ ID NO: 38 |
| B14 | NWGKYE | SEQ ID NO: 39 |
| B15 | NYGKYE | SEQ ID NO: 40 |
| B16 | NYAKYE | SEQ ID NO: 41 |
| B17 | NYCKYE | SEQ ID NO: 42 |
| B18 | NYDKYE | SEQ ID NO: 43 |
| B19 | NYEKYE | SEQ ID NO: 44 |
| B20 | NYFKYE | SEQ ID NO: 45 |
| B21 | NYGKYE | SEQ ID NO: 46 |
| B22 | NYHKYE | SEQ ID NO: 47 |
| B23 | NYIKYE | SEQ ID NO: 48 |
| B24 | NYKKYE | SEQ ID NO: 49 |
| B25 | NYLKYE | SEQ ID NO: 50 |
| C1 | NYMKYE | SEQ ID NO: 51 |
| C2 | NYNKYE | SEQ ID NO: 52 |
| C3 | NYPKYE | SEQ ID NO: 53 |
| C4 | NYQKYE | SEQ ID NO: 54 |
| C5 | NYRKYE | SEQ ID NO: 55 |
| C6 | NYSKYE | SEQ ID NO: 56 |
| C7 | NYTKYE | SEQ ID NO: 57 |
| C8 | NYVKYE | SEQ ID NO: 58 |
| C9 | NYWKYE | SEQ ID NO: 59 |
| C10 | NYYKYE | SEQ ID NO: 60 |
| C11 | NYGAYE | SEQ ID NO: 61 |
| C12 | NYGCYE | SEQ ID NO: 62 |
| C13 | NYGDYE | SEQ ID NO: 63 |
| C14 | NYGEYE | SEQ ID NO: 64 |
| C15 | NYGFYE | SEQ ID NO: 65 |
| C16 | NYGGYE | SEQ ID NO: 66 |
| C17 | NYGHYE | SEQ ID NO: 67 |
| C18 | NYGIYE | SEQ ID NO: 68 |
| C19 | NYGKYE | SEQ ID NO: 69 |
| C20 | NYGLYE | SEQ ID NO: 70 |
| C21 | NYGMYE | SEQ ID NO: 71 |
| C22 | NYGNYE | SEQ ID NO: 72 |
| C23 | NYGPYE | SEQ ID NO: 73 |
| C24 | NYGQYE | SEQ ID NO: 74 |
| C25 | NYGRYE | SEQ ID NO: 75 |
| D1 | NYGSYE | SEQ ID NO: 76 |
| D2 | NYGTYE | SEQ ID NO: 77 |
| D3 | NYGVYE | SEQ ID NO: 78 |
| D4 | NYGWYE | SEQ ID NO: 79 |
| D5 | NYGYYE | SEQ ID NO: 80 |
| D6 | NYGKAE | SEQ ID NO: 81 |
| D7 | NYGKCE | SEQ ID NO: 82 |
| D8 | NYGKDE | SEQ ID NO: 83 |
| D9 | NYGKEE | SEQ ID NO: 84 |
| D10 | NYGKFE | SEQ ID NO: 85 |
| D11 | NYGKGE | SEQ ID NO: 86 |
| D12 | NYGKHE | SEQ ID NO: 87 |
| D13 | NYGKIE | SEQ ID NO: 88 |
| D14 | NYGKKE | SEQ ID NO: 89 |
| D15 | NYGKLE | SEQ ID NO: 90 |
| D16 | NYGKME | SEQ ID NO: 91 |
| D17 | NYGKNE | SEQ ID NO: 92 |
| D18 | NYGKPE | SEQ ID NO: 93 |
| D19 | NYGKQE | SEQ ID NO: 94 |
| D20 | NYGKRE | SEQ ID NO: 95 |
| D21 | NYGKSE | SEQ ID NO: 96 |
| D22 | NYGKTE | SEQ ID NO: 97 |
| D23 | NYGKVE | SEQ ID NO: 98 |
| D24 | NYGKWE | SEQ ID NO: 99 |
| D25 | NYGKYE | SEQ ID NO: 100 |
| E1 | NYGKYA | SEQ ID NO: 101 |
| E2 | NYGKYC | SEQ ID NO: 102 |
| E3 | NYGKYD | SEQ ID NO: 103 |
| E4 | NYGKYE | SEQ ID NO: 104 |
| E5 | NYGKYF | SEQ ID NO: 105 |
| E6 | NYGKYG | SEQ ID NO: 106 |
| E7 | NYGKYH | SEQ ID NO: 107 |
| E8 | NYGKYI | SEQ ID NO: 108 |
| E9 | NYGKYK | SEQ ID NO: 109 |
| E10 | NYGKYL | SEQ ID NO: 110 |
| E11 | NYGKYM | SEQ ID NO: 111 |
| E12 | NYGKYN | SEQ ID NO: 112 |
| E13 | NYGKYP | SEQ ID NO: 113 |
| E14 | NYGKYQ | SEQ ID NO: 114 |
| E15 | NYGKYR | SEQ ID NO: 115 |
| E16 | NYGKYS | SEQ ID NO: 116 |
| E17 | NYGKYT | SEQ ID NO: 117 |
| E18 | NYGKYV | SEQ ID NO: 118 |
| E19 | NYGKYW | SEQ ID NO: 119 |
| E20 | NYGKYY | SEQ ID NO: 120 |
| E21 | NYGKYE | SEQ ID NO: 121 |
| E22 | NYGKYE | SEQ ID NO: 122 |
| E23 | NYGKYE | SEQ ID NO: 123 |
| E24 | NYGKYE | SEQ ID NO: 124 |
| E25 | NYGKYE | SEQ ID NO: 125 |

### Referenzliste

1. Benoit et al., Anal. Chem. 73 (2001), 2412-2420
2. Bowtell und Sambrook, In: DNA Microarrays: A cloning manual. Cold Spring Harbor Laboratory Press, Cold Spring Harbor, New York, (2003), ISBN 0-87969-624-9
3. Dittrich et al., Bioorg. Med. Chem. Lett. 8 (1998), 2351-2356
4. Dörwald, In: Organic Synthesis on Solid Phase, (2000) Wiley-VCH Verlag GmbH, Weinheim, Deutschland, ISBN 3-527-29950-5
5. Erck et al., Neurochem. Res. 25 (2000), 5-10
6. Frank, J. Immunol. Meth. 267 (2002), 13-26
7. Frank, Nucleic Acids Res. 11 (1983), 4365-4377
8. Frank, Tetrahedron 48 (1992), 9217-9232
9. Frank und Döring, Tetrahedron 44, 19 (1988), 6031-6040
10. Frank und Overwin, In: Methods in Molecular Biology, 66: Epitope Mapping Protocols (G. E. Morris, Ed.), The Humana Press Inc., Totowa, USA (1996), 149-169
11. Goddard et al., J. Chem. Soc. Chem. Commun. (1988), 1025-1027
12. Hansen et al., Int. J. Peptide Protein Res. 41 (1993), 237-245
13. Harlow und Lane, In: Antibodies: A laboratory manual. Cold Spring Harbor, New York, (1988)
14. Hoffmüller et al., Angew. Chem. Int. Ed. 38 (1999) 2000-2003
15. Sambrook et al., Molecular Cloning: A laboratory manual. Cold Spring Harbor, New York (1989), ISBN 0-87969-309-6
16. Southern et al., Nature Genetics Suppl. Nr. 1 (1999), 5-9
17. The SPOT-Synthesis Technique: Synthetic Peptide Arrays on Membrane Supports. In: Methods of parallel peptide synthesis and their contributions to deciphering molecular interactions in the immune system. Guest Editor: C. Granier, Part 3, The SPOT method of peptide synthesis: the role of arrayed peptides in revealing key features of antigen-antibody recognition. Special Issue of the Journal of Immunological Methods
18. Wölfl in: Transcript Laborwelt 3 (2000), 13-20
19. Xu et al., Molecular Diversity (2004), 1-10
20. Yershov et al., PNAS 93 (1996), 4913-4918

## Patentansprüche

1. Verfahren zur Herstellung eines Mikroarrays, wobei das Verfahren die Schritte umfasst:
(a) Zwei- bis mehrstufiges Synthetisieren von Sondenmolekülen an einem polymeren Träger, wobei Bindungen zwischen den Sondenmolekülen und dem polymeren Träger ausgebildet werden;
(b) Auflösen des polymeren Trägers mit den synthetischen Sondenmolekülen gemäß Schritt (a), wobei die Bindungen zwischen dem polymeren Träger und den Sondenmolekülen erhalten bleiben;
(c) Aufbringen der Lösung enthaltend den polymeren Träger mit den gebundenen Sondenmolekülen als Mikrotropfen auf eine planare Oberfläche.

2. Verfahren nach Anspruch 1, **gekennzeichnet durch** Reinigen der an den gemäß Schritt (b) aufgelösten polymeren Träger gebundenen Sondenmoleküle.

3. Verfahren nach Anspruch 1 oder Anspruch 2, **gekennzeichnet durch** Verdünnen der Lösung des polymeren Trägers mit den gebundenen Sondenmolekülen.

4. Verfahren nach einem der Ansprüche 1 bis 3, wobei die Bindungen zwischen den Sondenmolekülen und dem polymeren Träger chemische Bindungen, vorzugsweise kovalente Bindungen sind.

5. Verfahren nach einem der Ansprüche 1 bis 4, wobei sich nach Schritt (a) ein Schritt (a1) anschließt: (a1) Vorreinigen der Sondenmoleküle durch Waschen und gegebenenfalls Abspalten von Schutzgruppen.

6. Verfahren nach einem der Ansprüche 1 bis 5, wobei der polymere Träger in Schritt (b) in einer Mischung aus Trifluoressigsäure (TFA), Dichlormethan, Tri-isobutylsilan (TIBS) und Wasser aufgelöst wird.

7. Verfahren nach Anspruch 6, wobei die Mischung ca. 60-100 Vol.-% und insbesondere ca. 80-90 Vol.-% Trifluoressigsäure, vorzugsweise ca. 85 Vol.-%, ca. 0-15 Vol.-% und insbesondere ca. 5-15 Vol.-% Dichlormethan, vorzugsweise ca. 7 Vol.-%, ca. 0-15 Vol.% und insbesondere ca. 2-5 Vol.-% Tri-isobutylsilan, vorzugsweise ca. 3 Vol.-% und ca. 0-5 Vol.-% und insbesondere ca. 2-5 Vol.-% Wasser, vorzugsweise ca. 5 Vol.-% aufweist.

8. Verfahren nach einem der Ansprüche 1 bis 7, wobei die Reaktionszeit zum Auflösen des polymeren Trägers in Schritt (b) ca. 0,1-24 h, insbesondere ca. 2-24 h, vorzugsweise ca. 16 h beträgt.

9. Verfahren nach einem der Ansprüche 2 bis 8, wobei die Verbindungen durch Waschen in Diethylether, Aceton oder Tert-butylmethylether 3-5x, vorzugsweise 3x gereinigt werden.

10. Verfahren nach einem der Ansprüche 1 bis 9, wobei das polymere Trägermaterial mit den gebundenen Sondenmolekülen in Wasser, DMF, NMP oder DMSO, vorzugsweise DMSO, gelöst wird.

11. Verfahren nach einem der Ansprüche 1 bis 10, wobei die Sondenmoleküle kurzkettige Peptide, Polypeptide, Polysaccharide, Nukleinsäuren, DNA- oder RNA-Moleküle, oder insbesondere kleine organische Moleküle, sind.

12. Verfahren nach einem der Ansprüche 1 bis 11, wobei die planare Oberfläche in Schritt (c) die Oberfläche eines Mikrochips ist.

13. Verfahren nach einem der Ansprüche 1 bis 12, wobei die planare Oberfläche aus Metall, Glas, Plastik oder Keramik besteht.

14. Verfahren nach einem der Ansprüche 1 bis 13, wobei der polymere Träger Protein, Disulfid-vernetztes Polyacrylat oder Papier, insbesondere Cellulosepapier, ist.

15. Mikroarray für humane Biopsie, **gekennzeichnet durch**
- ein Material aus Metall, Glas, Plastik oder Keramik mit planarer Oberfläche
- mit darauf haftendem Präzipitat als eingetrocknete Mikrotropfen,
- wobei es sich bei dem Präzipitat um einen polymeren Träger mit daran kovalent gebundene Sondenmoleküle handelt, hergestellt nach einem Verfahren gemäß einem der vorhergehenden Ansprüche.

## Claims

1. Method of producing a microarray, the method comprising the following steps:
(a) synthesis, in two or more stages, of probe molecules on a polymeric support, bonds being formed between the probe molecules and the polymeric support;
(b) dissolving the polymeric support having the synthetic probe molecules in accordance with step (a), the bonds between the polymeric support and the probe molecules being retained;
(c) application of the solution containing the polymeric support having the bound probe molecules in the form of microdrops to a planar surface.

2. Method according to claim 1, **characterised in** purification of the probe molecules bound to the dissolved polymeric support according to Step (b).

3. Method according to claim 1 or claim 2, **characterised in** dilution of the solution of the polymeric support having the bound probe molecules.

4. Method according to any one of claims 1 to 3, the bonds between the probe molecules and the polymeric support being chemical bonds, preferably covalent bonds.

5. Method according to any one of claims 1 to 4, Step (a) being followed by a Step (a1): (a1) pre-purification of the probe molecules by washing and optionally removal of protecting groups.

6. Method according to any one of claims 1 to 5, the polymeric support in Step (b) being dissolved in a mixture of trifluoroacetic acid (TFA), dichloromethane, tri-isobutylsilane (TIBS) and water.

7. Method according to claim 6, the mixture containing about 60-100 % by vol. and especially about 80-90 % by vol., trifluoroacetic acid, preferably about 85 % by vol., about 0-15 % by vol. and especially about 5-15 % by vol., dichloromethane, preferably about 7 % by vol., about 0-15 % by vol. and especially about 2-5 % by vol., tri-isobutylsilane, preferably about 3 % by vol., and about 0-5 % by vol. and especially about 2-5 % by vol., water, preferably about 5 % by vol.

8. Method according to any one of claims 1 to 7, the reaction time for dissolving the polymeric support in Step (b) being about 0.1 - 24 hours, especially about 2 - 24 hours, preferably about 16 hours.

9. Method according to any one of claims 2 to 8, the compounds being purified by washing 3-5 x, preferably 3 x, in diethyl ether, acetone or tert-butylmethyl ether.

10. Method according to any one of claims 1 to 9, the polymeric support material having the bound probe molecules being dissolved in water, DMF, NMP or DMSO, preferably DMSO.

11. Method according to any one of claims 1 to 10, the probe molecules being short-chain peptides, polypeptides, polysaccharides, nucleic acids, DNA molecules or RNA molecules, or especially small organic molecules.

12. Method according to any one of claims 1 to 11, the planar surface in Step (c) being the surface of a microchip.

13. Method according to any one of claims 1 to 12, the planar surface consisting of metal, glass, plastics material or ceramics.

14. Method according to any one of claims 1 to 13, the polymeric support being protein, disulfide-crosslinked polyacrylate or paper, especially cellulose paper.

15. Microarray for a human biopsy, **characterised by**
- a material from metal, glass, plastics material or ceramics having a planar surface
- with a precipitate in the form of dried microdrops adhering thereon,
- wherein the precipitate is a polymeric support having probe molecules that are covalently bound at it,
produced in accordance with a method of any one of the preceding claims.

## Revendications

1. Procédé de fabrication d'un microréseau, le procédé comprenant les étapes consistant à :
(a) synthétiser en deux étapes ou plus des molécules sondes sur un support polymère, où des liaisons sont formées entre les molécules sondes et le support polymère ;
(b) solubiliser le support polymère avec les molécules sondes synthétiques selon l'étape (a), où les liaisons entre les molécules sondes et le support polymère sont conservées ;
(c) appliquer sous forme de microgouttes la solution contenant le support polymère avec les molécules sondes liées sur une surface plane.

2. Procédé selon la revendication 1, **caractérisé par** la purification des molécules sondes liées au support polymère solubilisé selon l'étape (b).

3. Procédé selon la revendication 1 ou 2, **caractérisé par** la dilution de la solution du support polymère avec les molécules sondes liées.

4. Procédé selon l'une des revendications 1 à 3, dans lequel les liaisons entre les molécules sondes et le support polymère sont des liaisons chimiques, de préférence des liaisons covalentes.

5. Procédé selon l'une des revendications 1 à 4, dans lequel une étape (a1), consistant à :
(a1) purifier au préalable les molécules sondes par lavage et le cas échéant clivage des groupes protecteurs,
est adjointe à l'étape (a).

6. Procédé selon l'une des revendications 1 à 5, dans lequel le support polymère est solubilisé à l'étape (b) dans un mélange constitué d'acide trifluoroacétique (TFA), de dichlorométhane, de tri-isobutylsilane (TIBS) et d'eau.

7. Procédé selon la revendication 6, dans lequel le mélange comprend environ 60 à 100 % en volume et notamment environ 80 à 90 % en volume d'acide trifluoroacétique, de préférence environ 85 % en volume, environ 0 à 15 % en volume et en particulier environ 5 à 15 % en volume de dichlorométhane, de préférence environ 7 % en volume, environ 0 à 15 % en volume et en particulier environ 2 à 5 % en volume de tri-isobutylsilane, de préférence environ 3 % en volume et environ 0 à 5 % en volume et en particulier environ 2 à 5 % en volume d'eau, de préférence environ 5 % en volume.

8. Procédé selon l'une des revendications 1 à 7, dans lequel le temps de réaction pour la solubilisation du support polymère à l'étape (b) atteint environ 0,1 à 24 heures, en particulier environ 2 à 24 h, de préférence environ 16 heures.

9. Procédé selon l'une des revendications 2 à 8, dans lequel les composés sont purifiés par lavage dans du diéthyléther, de l'acétone ou du tert.-butylméthyléther 3 à 5 fois, de préférence 3 fois.

10. Procédé selon l'une des revendications 1 à 9, dans lequel le matériau support polymère avec les molécules sondes qui y sont liées est dissous dans l'eau, le DMF, la NMP ou le DMSO, de préférence le DMSO.

11. Procédé selon l'une des revendications 1 à 10, dans lequel les molécules sondes sont des peptides à chaînes courtes, des polypeptides, des polysaccharides, des acides nucléiques, des molécules d'ADN ou d'ARN, ou en particulier des petites molécules organiques.

12. Procédé selon l'une des revendications 1 à 11, dans lequel la surface plane à l'étape (c) est la surface d'une micropuce.

13. Procédé selon l'une des revendications 1 à 12, dans lequel la surface plane se compose de métal, de verre, de plastique ou de céramique.

14. Procédé selon l'une des revendications 1 à 13, dans lequel le support polymère est une protéine, un polyacrylate réticulé par du disulfure ou du papier, en particulier un papier cellulosique.

15. Microréseau pour biopsie humaine, **caractérisé par**
- une matière en métal, verre, plastique ou céramique avec une surface plane
- avec un précipité qui y adhère sous forme de microgouttes séchées,
- dans lequel le précipité est un support polymère avec des molécules sondes qui y sont liées de manière covalente,
préparé d'après un procédé selon l'une des revendications précédentes.
